# EUROPEAN PATENT SPECIFICATION

(11) **EP 1 153 616 B1**
(45) Date of publication and mention of the grant of the patent: **01.12.2004**
(21) Application number: 00902958.8
(22) Date of filing: 14.02.2000
(51) Int. Cl.: A61K 47/36, A61K 47/10, A61K 47/26, A61K 9/20

(54) **TABLETS QUICKLY DISINTEGRATED IN THE ORAL CAVITY**
IM MUNDRAUM SCHNELLZERFALLENDE TABLETTEN
COMPRIMES SE DESINTEGRANT RAPIDEMENT DANS LA CAVITE BUCCALE

(30) Priority: 15.02.1999 JP 3542999
(43) Date of publication of application: 14.11.2001
(73) Proprietor: Sumitomo Pharmaceuticals Company, Limited, Osaka-shi, Osaka 541-8510 (JP)
(72) Inventor: NISHII, Hiroyuki, Takatsuki-shi, Osaka 569-1029 (JP); KOBAYASHI, Hirohisa, Ibaraki-shi, Osaka 567-0824 (JP); OTODA, Kazuya, Takarazuka-shi, Hyogo 665-0877 (JP)
(74) Representative: Cresswell, Thomas Anthony
(86) International application number: PCT/JP2000/000806
(87) International publication number: WO 2000/047233

(56) References cited:
- EP-A- 1 022 021
- EP-A2- 0 553 777
- WO-A-99/18936
- GB-A- 1 336 609
- GB-A- 1 439 240
- JP-A- 4 091 029
- JP-A- 8 143 473
- JP-A- 8 208 523
- JP-A- 9 095 447
- JP-A- 10 298 062

## Description

### TECHNICAL FIELD

This invention relates to a quick-disintegrating tablet preparation exhibiting rapid disintegratability or solubility in the mouth with little water or even without water.

### BACKGROUND ART

Heretofore known is a variety of oral dosage forms but few reflect sufficient attention to the ease of taking by the patient and there is a demand for dosage forms suited to the aged, children, and seriously ill patients who are frequently poor in compliance. For example, tablets and capsules are the dosage forms used most universally in view of the accuracy of dosage, physicochemical stability, and even the cost of manufacture but, among patients, not a few dislike those dosage forms because of their untoward mouth-feel and tendencies toward getting caught in the throat. Powders and granules may not be completely swallowed, leaving residues in the mouth and giving a lingering unpleasant after-taste. It is acknowledged that liquid dosage forms such as syrups are suitable for both the aged and children but divided dosing from a graduated bottle is not only onerous to geriatric, pediatric, or seriously ill patients but cannot be expected to insure the accuracy of dosage. Drawbacks in terms of physicochemical stability have also been noted.

Recent years have witnessed the development of many technologies directed to the pharmaceutical dosage form which would disintegrate quickly in the mouth and have both the accuracy of dosage and physicochemical stability of tablets and capsules and the ease of ingestion of syrups in one [JP Kokai H9-309821, JP Kokai H9-309822, WO93/12769, JP Kohyo H7-501829].

However, the above-mentioned technologies are not satisfactory in assuring the stability of medicaments because comparatively large amounts of water must be used or are deficient in assuring a sufficient product strength to resist damage in the course of distribution. Furthermore, some products have difficulties in handling, while others require a complicated manufacturing process or a special production equipment. Technologies suited for more universal exploitation have been disclosed in JP Kokai H10-182436 and JP Kokai H9-71523, for instance, but there is still room for improvement in the quick disintegratability in the mouth.

### DISCLOSURE OF INVENTION

The object of this invention is to provide a tablet preparation which can be easily manufactured by means of the conventional production equipment and without requiring any extraordinary pharmaceutical expertise, insures quick disintegratability in the mouth and good sensory acceptability and yet has a suitable degree of strength to resist damage in the course of distribution.

The inventors did intensive investigations for accomplishing the above object and found that by using a starch and a water-soluble excipient in combination as the formulation additive, tablets adapted to disintegrate in the mouth within 1 minute and yet having practically useful hardness can be provided notwithstanding the common belief that such tablets can hardly be manufactured with the conventional compression machine. The finding led to the development of this invention. In particular, the effect derived from the formulation of starch in terms of disintegratability and sensory acceptability is quite pronounced as compared with the use of the conventional disintegrators such as low-substitution-degree hydroxypropylcellulose, carboxyethylcellulose calcium, crospovidone, etc. Indeed, this was a surprising finding which could never be anticipated from the state-of-the art information.

This invention, therefore, relates to the following tablet preparations.
[1] A tablet preparation characterized by its comprising a starch, a water-soluble excipient, a lubricant and a medicament and substantially not containing a binder other than starch, wherein the water-soluble excipient is at least one member selected from mannitol and lactose, and the lubricant is at least one member selected from magnesium stearate, calcium stearate, sodium stearyl fumarate and light silicic anhydride.
[2] The above-mentioned tablet preparation [1] wherein the starch is at least one member selected from corn starch, potato starch, wheat starch and rice starch.
[3] The above-mentioned tablet preparation [1] or [2] wherein the water-soluble excipient is mannitol.
[4] The above-mentioned tablet preparation [1]-[3] wherein the total amount of starch and water-soluble excipient is not less than 50%.
[5] The above-mentioned tablet preparation [1]-[4] wherein the amount of the medicament is not more than 50%.
[6] The above-mentioned tablet preparation [1]-[5] wherein the formulating amount of starch with respect to the total amount of starch and water-soluble excipient is not less than 5%.
[7] The above-mentioned tablet preparation [1]-[6] wherein the formulating amount of starch with respect to the total amount of starch and water-soluble excipient is 5-50%.

For use in this invention, the starch includes various starches which can be formulated in pharmaceutical products, such as corn starch, potato starch, wheat starch and rice starch. The water-soluble excipient is mannitol or lactose, preferably mannitol. The lubricant is magnesium stearate, calcium stearate, sodium stearyl fumarate or light silicic anhydride, preferably magnesium stearate or sodium stearyl fumarate.

The active ingredient or medicament is not particularly restricted insofar as it is intended for oral administration but includes, to mention a few examples of medicaments to which this invention can be applied with particular advantage, antipyretic-antiinflammatory agents such as indomethacin, ibuprofen, ketoprofen, acetaminophen, aspirin, and isopropylantipyrine; antihistaminics such as diphenylpyraline hydrochloride, chlorpheniramine maleate, cimetidine and isothipendyl hydrochloride ; cardiovascular drugs such as phenylephrine hydrochloride, procainamide hydrochloride, quinidine sulfate, and isosorbide dinitrate; antihypertensives such as amlodipine besylate, arotinolol hydrochloride, tranquilizers such as sulpiride, diazepam, valproic acid, lithium carbonate, tandospirone citrate, etc.; antibiotics such as cefalesin, and ampicillin; peptides or proteins such as insulin, vasopressin, interferon, interleukin-2, urokinase, and various growth factors, e.g. human growth hormone ; and other drugs such as theophylline, caffein, carbetapentane citrate, phenylpropanolamine hydrochloride, etidronate disodium, cetirizine hydrochloride, and droxidopa.

The tablet preparation of this invention does not contain a binder other than starch in any substantial amount, for binders other than starch detract from the effect of this invention, namely attainment of tablets which disintegrate in the mouth substantially within one minute. The binder other than starch which is substantially not contained in the preparation of this invention includes but is not limited to polyvinyl alcohol, polyvinylpyrrolidone, hydroxypropylmethylcellulose, hydroxypropylcellulose, agar and gelatin.

The pharmaceutical preparation of this invention may contain, in addition to said ingredients, various nontoxic, inert additives which are in routine use in the pharmaceutical field. As such additives, various substances which will not interfere with the effect of this invention and are generally used as pharmaceutical additives can be mentioned. For example, excipients such as xylitol, sorbitol, trehalose, glucose, sucrose, talc, kaolin, calcium hydrogenphosphate, calcium sulfate, calcium carbonate, crystalline cellulose; disintegrators such as carboxymethylcellulose calcium, low-substitution-degree hydroxymethylcellulose; and other formulation additives such as corrigents, antiseptics, stabilizers, antistatic agents, effervescent agents and coloring agents.

High-sweetness artificial sweeteners such as aspartame, saccharin sodium, stevia, etc. and flavoring agents such as peppermint, spearmint, menthol, lemon, orange, grapefruit, pine, fruit, yogurt, etc. can also be incorporated, and may contribute to a more favorable sensory acceptability in some instances.

The mean particle diameter of said starch, water-soluble excipient or medicament is not particularly restricted but as far as the water-soluble excipient is concerned, the range of 10-500 µm is preferred and that of 30-200 µm is still more preferred. If the particle diameter is too large, compressibility will be adversely affected so that the tablet strength tends to decrease. Conversely if the particle diameter is too small, disintegratability will be seriously compromised, although improvements in compressibility will be obtained.

The total content of the starch and water-soluble excipient in the preparation is preferably not less than 50%, more preferably not less than 70%.

The formulating amount of the medicament or active ingredient depends on the kind of active ingredient but is usually not more than 50%, preferably not more than 30%, more preferably not more than 10%.

The formulating amount of starch based on the total content of starch and water-soluble excipient is not less than 5%, preferably 5-50%, more preferably 5-30%.

While the manufacturing technology for the tablet preparation of this invention is not particularly restricted, tablets may be manufactured by the following process.

Tablets can be obtained by blending the starch, water-soluble excipient and medicament and compressing the resulting composition. When the content uniformity of the medicament may hardly be assured because of the cohesive nature or large crystal sizes of the ingredients used, it is preferable to carry out a size alignment by a suitable technique such as milling before or after blending so that an adequate content uniformity may be assured. As an alternative, where necessary, the composition may be granulated in advance and compressed.

The tablet-forming technology is not particularly restricted but a compression tableting method using a rotary tablet machine, a single-punch tablet machine, or a hydraulic press can be employed. The compressive pressure to be applied is not particularly restricted insofar as sufficient strength may be imparted to the product tablet but is preferably not less than 50 kg.

The shape of the tablet which can be provided in accordance with this invention is not particularly restricted but may be any of discoid, discoid R-edge, round bevel-edge, and various irregular shapes, and may be scored for breaking.

The following examples illustrate this invention in further detail.

### Example 1

| | |
|---|---|
| Tandospirone citrate | 5 mg |
| D-mannitol | 103 mg |
| Corn starch | 11 mg |
| Magnesium stearate | 1 mg |

The above ingredients were blended and compressed with a hydraulic press (manufactured by Riken) at a pressure of 50 kgf to provide a tablet preparation measuring 7 mm in diameter and weighing 120 mg.

### Example 2

| | |
|---|---|
| Tandospirone citrate | 5 mg |
| D-mannitol | 90 mg |
| Corn starch | 24 mg |
| Magnesium stearate | 1 mg |

The above ingredients were blended and compressed with a hydraulic press (manufactured by Riken) at a pressure of 50 kgf to provide a tablet preparation measuring 7 mm in diameter and weighing 120 mg.

### Example 3

| | |
|---|---|
| Tandospirone citrate | 10 mg |
| Lactose | 150 mg |
| Wheat starch | 37 mg |
| Sodium stearyl fumarate | 3 mg |

The above ingredients were blended and compressed with a hydraulic press (manufactured by Riken) at a pressure of 50 kgf to provide a tablet preparation measuring 8 mm in diameter and weighing 200 mg.

### Example 4

| | |
|---|---|
| Arotinolol hydrochloride | 5 mg |
| D-mannitol | 101.9 mg |
| Corn starch | 11 mg |
| Aspartame | 1 mg |
| Flavoring agent | 0.1 mg |
| Sodium stearyl fumarate | 1 mg |

The above ingredients were blended and compressed with a hydraulic press (manufactured by Riken) at a pressure of 50 kgf to provide a tablet preparation measuring 7 mm in diameter and weighing 120 mg.

### Example 5

| | |
|---|---|
| Amlodipine besylate | 5 mg |
| D-mannitol | 103 mg |
| Corn starch | 11 mg |
| Magnesium stearate | 1 mg |

The above ingredients were blended and compressed with a hydraulic press (manufactured by Riken) at a pressure of 50 kgf to provide a tablet preparation measuring 7 mm in diameter and weighing 120 mg.

### Example 6

| | |
|---|---|
| Amlodipine besylate | 5 mg |
| D-mannitol | 91 mg |
| Corn starch | 23 mg |
| Magnesium stearate | 1 mg |

The above ingredients were blended and compressed with a hydraulic press (manufactured by Riken) at a pressure of 50 kgf to provide a tablet preparation measuring 7 mm in diameter and weighing 120 mg.

### Example 7

| | |
|---|---|
| Amlodipine besylate | 5 mg |
| D-mannitol | 101.9 mg |
| Corn starch | 11 mg |
| Aspartame | 1 mg |
| Flavoring agent | 0.1 mg |
| Sodium stearyl fumarate | 1 mg |

The above ingredients were blended and compressed with a hydraulic press (manufactured by Riken) at a pressure of 50 kgf to provide a tablet preparation measuring 7 mm in diameter and weighing 120 mg.

### Example 8

| | |
|---|---|
| Amlodipine besylate | 45 g |
| D-mannitol | 869.4 g |
| Corn starch | 96.3 g |
| Low-substitution-degree | |
| hydroxypropylcellulose | 32.4 g |
| Aspartame | 9 g |
| Light silicic anhydride | 5.4 g |
| Flavoring agent | 0.9 g |
| Sodium stearyl fumarate | 21.6 g |

A portion (6.3 g) of corn starch, among the above ingredients, was taken and dispersed in purified water, and the dispersion was gelatinized by warming to prepare 630 g of 1% starch size. A spray granulator (RABO-1, manufactured by Paulex) was charged with amlodipine besylate, D-mannitol, the remainder (90 g) of corn starch, low-substitution-degree hydroxypropylcellulose, aspartame and light silicic anhydride, followed by mixing, and the 1% starch size was added. The granulation was dried and transferred to a V-mixer (Model S-5, manufactured by Tsutsui Rikagaku) in which the flavoring agent and sodium stearyl fumarate were added and mixed to provide a granulation for compression tableting. This granulation was compressed with a rotary tablet machine (HT-P15A, manufactured by Hata Iron Works) at a pressure of 400 kg to provide about 9000 tablets each measuring 7 mm in diameter and weighing 120 mg.

### Comparative Example 1

| | |
|---|---|
| Tandospirone citrate | 5 mg |
| D-mannitol | 114 mg |
| Magnesium stearate | 1 mg |

The above ingredients were blended and compressed with a hydraulic press (manufactured by Riken) at a pressure of 50 kgf to provide a tablet preparation measuring 7 mm in diameter and weighing 120 mg.

### Comparative Example 2

| | |
|---|---|
| Amlodipine besylate | 5 mg |
| D-mannitol | 114 mg |
| Magnesium stearate | 1 mg |

The above ingredients were blended and compressed with a hydraulic press (manufactured by Riken) at a pressure of 50 kgf to provide a tablet preparation measuring 7 mm in diameter and weighing 120 mg.

### Test Example 1

The tablets manufactured in Examples 1, 2, 5, 6 and 8 and Comparative Examples 1 and 2 were respectively tested for disintegration time and hardness with a mechanical disintegratability tester (Model NT-6H, Toyama Sangyo K.K.) and a mechanical tablet strength tester (TH-203CP, Toyama Sangyo K.K).

Furthermore, these tablets were respectively administered into the oral cavity and the time to complete disintegration by saliva alone in the mouth was measured and recorded as oral disintegration time. Furthermore, the sensory acceptability was evaluated on the 3-grade scale of ○: good, Δ: neither good nor bad, ×: bad.

The results are shown in Table 1. Thus, the tablets according to Example 1, 2, 5, 6 and 8 were invariably satisfactory in hardness and disintegration time and rated good in sensory acceptability. On the other hand, the tablets according to Comparative Example 1 were rather satisfactory in hardness but were remarkably retarded in disintegration and rated low in sensory acceptability.

The tablets of Comparative Example 2 were also slightly low in hardness, showed a long disintegration time, and was rated poor in sensory acceptability.

**Table 1**

| | Example 1 | Example 2 | Example 5 | Example 6 | Example 8 | Compar. Ex. 1 | Compar. Ex. 2 |
|---|---|---|---|---|---|---|---|
| Hardness (kg) | 4 | 3 | 4 | 5 | 3 | 3 | 2 |
| Disintegration time (sec) | 19 | 27 | 15 | 35 | 30 | 411 | 80 |
| Oral disintegration time (sec) | 20 | 21 | 17 | 33 | 14 | >240 | 100 |
| Sensory acceptability | ○ | ○ | ○ | ○ | ○ | × | × |

### EFFECTS OF INVENTION

Tablets having sufficient hardness to resist damage in the course of distribution and yet disintegrating rapidly in the oral cavity can be manufactured without resort to any extraordinary pharmaceutical expertise. As a result, elderly as well as pediatric patients who are handicapped in swallowing power are now enabled to take medicines easily even without the aid of water.

## Claims

1. A tablet preparation **characterized by** its comprising a starch, a water-soluble excipient, a lubricant and a medicament and substantially not containing a binder other than starch, wherein the water-soluble excipient is at least one member selected from mannitol and lactose, and the lubricant is at least one member selected from magnesium stearate, calcium stearate, sodium stearyl fumarate and light silicic anhydride.

2. A tablet preparation as defined in claim 1 wherein the starch is at least one member selected from corn starch, potato starch, wheat starch and rice starch.

3. A tablet preparation as defined in claim 1 or 2 wherein the water-soluble excipient is mannitol.

4. A tablet preparation as defined in any of claims 1-3 wherein the total amount of starch and water-soluble excipient is not less than 50%.

5. A tablet preparation as defined in any of claims 1-4 wherein the amount of the medicament is not more than 50%.

6. A tablet preparation as defined in any of claims 1-5 wherein the formulating amount of starch with respect to the total amount of starch and water-soluble excipient is not less than 5%.

7. A tablet preparation as defined in any of claims 1-6 wherein the formulating amount of starch with respect to the total amount of starch and water-soluble excipient is 5-50%.

8. A tablet preparation as defined in any of claims 1-7 wherein the medicament is tandospirone citrate, arotinolol hydrochloride or amlodipine besylate.

## Patentansprüche

1. Tablettenzubereitung, die **dadurch gekennzeichnet ist, dass** sie eine Stärke, einen wasserlöslichen Excipienten, ein Gleitmittel und ein Arzneimittel umfasst und die im Wesentlichen kein Bindemittel außer Stärke enthält, wobei der wasserlösliche Excipient mindestens ein Vertreter, ausgewählt aus Mannitol und Lactose, ist und das Gleitmittel mindestens ein Vertreter, ausgewählt aus Magnesiumstearat, Calciumstearat, Natriumstearylfumarat und leichtem Kieselsäureanhydrid, ist.

2. Tablettenzubereitung, die wie in Anspruch 1 definiert ist, wobei die Stärke mindestens ein Vertreter, ausgewählt aus Maisstärke, Kartoffelstärke, Weizenstärke und Reisstärke, ist.

3. Tablettenzubereitung, die wie in Anspruch 1 oder 2 definiert ist, wobei der wasserlösliche Excipient Mannitol ist.

4. Tablettenzubereitung, die wie in einem der Ansprüche 1-3 definiert ist, wobei die Gesamtmenge an Stärke und wasserlöslichem Excipienten nicht weniger als 50% beträgt.

5. Tablettenzubereitung, die wie in einem der Ansprüche 1-4 definiert ist, wobei die Menge des Arzneimittels nicht mehr als 50% beträgt.

6. Tablettenzubereitung, die wie in einem der Ansprüche 1-5 definiert ist, wobei die formulierende Menge an Stärke im Hinblick auf die Gesamtmenge an Stärke und wasserlöslichem Excipienten nicht weniger als 5% beträgt.

7. Tablettenzubereitung, die wie in einem der Ansprüche 1-6 definiert ist, wobei die formulierende Menge an Stärke im Hinblick auf die Gesamtmenge an Stärke und wasserlöslichem Excipienten 5-50% beträgt.

8. Tablettenzubereitung, die wie in einem der Ansprüche 1-7 definiert ist, wobei das Arzneimittel Tandospironcitrat, Arotinololhydrochlorid oder Amlodipinbesilat beträgt.

## Revendications

1. Préparation pour comprimé **caractérisée en ce qu'**elle comprend un amidon, un excipient soluble dans l'eau, un lubrifiant et un médicament, et ne contenant substantiellement pas de liant autre que l'amidon, dans laquelle l'excipient soluble dans l'eau est au moins un élément choisi parmi le mannitol et le lactose, et le lubrifiant est au moins un élément choisi parmi le stéarate de magnésium, le stéarate de calcium, le stéarylfumarate de sodium et l'anhydride silicique léger.

2. Préparation pour comprimé selon la revendication 1, dans laquelle l'amidon est au moins un élément choisi parmi l'amidon de maïs, l'amidon de pomme de terre, l'amidon de blé et l'amidon de riz.

3. Préparation pour comprimé selon la revendication 1 ou 2, dans laquelle l'excipient soluble dans l'eau est le mannitol.

4. Préparation pour comprimé selon l'une quelconque des revendications 1 à 3, dans laquelle la quantité totale d'amidon et d'excipient soluble dans l'eau n'est pas inférieure à 50 %.

5. Préparation pour comprimé selon l'une quelconque des revendications 1 à 4, dans laquelle la quantité de médicament n'est pas supérieure à 50 %.

6. Préparation pour comprimé selon l'une quelconque des revendications 1 à 5, dans laquelle la quantité d'amidon de la formulation, par rapport à la quantité totale d'amidon et d'excipient soluble dans l'eau, n'est pas inférieure à 5 %.

7. Préparation pour comprimé selon l'une quelconque des revendications 1 à 6, dans laquelle la quantité d'amidon de la formulation, par rapport à la quantité totale d'amidon et d'excipient soluble dans l'eau, est de 5 à 50 %.

8. Préparation pour comprimé selon l'une quelconque des revendications 1 à 7, dans laquelle le médicament est le citrate de tandospirone, le chlorhydrate d'arotinolol ou le bésylate d'amlodipine.
